# FASCICULE DE BREVET EUROPEEN

(11) **EP 0 722 496 B1**
(45) Date de publication et mention de la délivrance du brevet: **30.11.2005**
(21) Numéro de dépôt: 94928933.4
(22) Date de dépôt: 29.09.1994
(51) Int. Cl.: C12N 15/11, C07K 14/82, A61K 31/70, C12N 7/01, C12N 15/86

(54) **COMPOSITIONS PHARMACEUTIQUES ET LEUR UTILISATION, NOTAMMENT POUR LE TRAITEMENT DES MALADIES NEURODEGENERATIVES**
PHARMAZEUTISCHE ZUSAMMENSETZUNGEN UND DEREN VERWENDUNG INSBESONDERE IN DER BEHANDLUNG VON NEURODEGENERATIVEN KRANKHEITEN
PHARMACEUTICAL COMPOSITIONS AND UTILIZATION THEREOF PARTICULARLY FOR THE TREATMENT OF NEURODEGENERATIVE DISEASES

(30) Priorité: 04.10.1993 FR 9311774
(43) Date de publication de la demande: 24.07.1996
(60) Demande divisionnaire: 05016159.5
(73) Titulaire: Aventis Pharma S.A., 92160 Antony (FR)
(72) Inventeur: MALLET, Jacques, F-75013 Paris (FR); REVAH, Frédéric, F-92160 Antony (FR); STUTZMANN, Jean-Marie, F-94440 Villecresnes (FR)
(74) Mandataire: Savina, Jacques
(86) Numéro de dépôt international: PCT/FR1994/001142
(87) Numéro de publication internationale: WO 1995/009916

(56) Documents cités:
- WO-A-91/18088
- WO-A-92/05272
- WO-A-92/19732
- PROCEEDINGS OF THE NATIONAL ACADEMY OF SCIENCES OF USA, vol.90, no.8, 15 Avril 1993, WASHINGTON US pages 3319 - 3323 FRIEDMAN, P. ET AL. 'The p53 protein is an unusually shaped tetramer that binds directly to DNA' cité dans la demande
- JOURNAL OF CELLULAR BIOCHEMISTRY, vol.45, no.1, Janvier 1991, NEW YORK, USA pages 22 - 29 MICHALOVITZ, D. ET AL. 'p53 mutations: gains or losses ?' cité dans la demande
- SCIENCE, vol.252, 21 Juin 1991, LANCASTER, PA US pages 1708 - 1711 KERN, S. ET AL. 'Identification of p53 as a sequence-specific DNA-binding protein' cité dans la demande

## Description

La présente invention concerne des compositions pharmaceutiques et leur utilisation, notamment pour le traitement des maladies neurodégénératives. Elle concerne plus particulièrement l'utilisation de composés agissant sur la protéine p53 ou sur son gène pour la préparation d'une composition pharmaceutique destinée au traitement des maladies neurodégénératives.
Le gène p53 code pour une protéine nucléaire de 53 kDa. Le gène sauvage codant pour la p53 native possède une activité anti-oncogène [pour revue, voir par exemple Oren, FASEB J. 6 (1992) 3169]. En particulier, la protéine p53 sauvage est capable d'inhiber la formation des foyers de transformation dans des fibroblastes de rongeurs transfectés avec diverses combinaisons d'oncogènes. La forme mutée par délétion et/ou mutation de ce gène est au contraire impliquée dans le développement de la plupart des cancers humains [Baker et coll., Science 244 (1989) 217]. Ses formes mutées sont également capables de coopérer avec les oncogènes ras pour transformer des fibroblastes murins. Pour cette raison, la protéine p53 ou son gène ont été largement étudiés en tant que cibles pour le traitement des cancers. Par ailleurs, Chopp et al. (Biochem.Biophys.Res.Com 182 (1992) 1201) ont décrit une expression de p53 dans le cerveau de rat ischémié. Cependant, rien n'indique dans ces résultats si cette expression constitue une cause de la neurodégénérescence, ou un phénomène parallèle. De plus, aucune approche thérapeutique n'est envisagée ni suggérée dans ce document.

La présente invention résulte en partie de la mise en évidence que la protéine p53 constitue un médiateur de la dégénérescence neuronale. Elle résulte également de la mise en évidence que l'emploi de composés capables d'inhiber au moins partiellement l'activité de la protéine p53 peut permettre de bloquer le processus de mort neuronale.

Pour étudier les mécanismes moléculaires de la dégénérescence neuronale, la demanderesse a utilisé comme modèle des souris chez lesquelles l'expression du gène p53 a été inactivée [Donehower et al., Nature 356 (1992) 215]. Des expériences d'ischémie focale irréversible ont été pratiquées sur ces souris, et les volumes d'infarct ont été comparées avec celles observées chez des souris sauvages controle (même souche, même sexe, même âge, même fournisseur). Les résultats obtenus ont montré une diminution statistiquement significative de 20 % des volumes d'infarct après ischémie des souris n'exprimant pas le gène p53 (Cf exemples). De plus, la demanderesse a également démontré que l'utilisation d'antisens anti-p53 permet de ralentir la mort induite par le glutamate sur des cultures de cellules corticales. Ces résultats démontrent que la protéine p53 joue un role de médiateur de la dégénérescence neuronale, observation qui n'a jamais été rapportée dans l'art antérieur, et qu'un controle de l'activité de cette protéine permet de lutter contre la mort neuronale. La protéine p53, son gène et l'ensemble des facteurs susceptibles d'interagir avec elle constituent donc de nouvelles cibles pharmacologiques dans le traitement des processus neurodégénératifs. L'invention réside donc en partie dans l'utilisation de composés capables de bloquer au moins partiellement l'activité de p53 pour le traitement des maladies neurodégénératives.

Un premier objet de la présente invention réside dans l'utilisation d'un composé inhibant au moins partiellement l'activité de la protéine p53 pour la préparation d'une composition pharmaceutique destinée au traitement et/ou à la prévention des maladies neurodégénératives.
Les composés inhibant au moins partiellement l'activité de la protéine p53 au sens de la présente invention peuvent être des composés agissant (i) sur la synthèse de p53, aux niveaux transcriptionnel, traductionnel ou post-traductionnel, ou (ii) sur la liaison de p53 à l'ADN.

Parmi les composés agissant sur la synthèse de la protéine p53, on peut citer les séquences nucléotidiques antisens capable de réduire ou de supprimer l'expression de p53, au niveau transcriptionnel ou traductionnel.
De telles séquences peuvent en effet être dirigées contre l'ARNm de p53 et agir sur la traduction de celui-ci en protéine : il peut s'agir d'oligonucléotides (synthétiques, modifiés chimiquement, etc tels que décrits par exemple dans les demandes EP 092 574, EP 231 495, WO 92/03568; WO 91/13080, etc) ou de séquences d'ADN codant pour des ARN capables d'interagir sélectivement avec l'ARNm de p53, selon la technique décrite par exemple dans la demande EP 140 308.
De telles séquences peuvent également être dirigées contre le gène codant pour p53 et agir sur la transcription de celui-ci en ARN. Plus particulièrement, ces séquences peuvent être dirigées contre des régions codantes du gène (gène de structure de p53), ou contre des régions non codantes : régions régulatrices de la transcription, exons, etc. De telles séquences peuvent être préparées dans les conditions décrites par exemple dans EP 558 634, WO 91/06626, WO92/10590, WO 93/10820, etc).

Parmi les composés agissant sur la liaison de p53 à l'ADN, on peut citer plus particulièrement des antagonistes de p53, ou des protéines capables d'interagir avec p53 et de moduler ainsi son activité de laison à l'ADN. A cet égard, on peut citer les mutants dominants négatifs de p53 constitués essentiellement de forme mutée inactive, qui sont capables d'entrer en compétition avec la protéine sauvage pour l'interaction avec l'ADN. De tels mutants sont par exemple le mutant p53Va1135, ou d'autres formes décrites par exemple dans Michalovitz et al. [J. Cell. Bioch. 45 (1991) 22]. Ils peuvent être utilisés tels quels, mais, préférentiellement, ils sont utilisés dans le cadre de la présente invention sous forme de constructions génétiques capables d'exprimer in vivo ces mutants. D'autres composés capables d'inhiber au moins partiellement la liaison de p53 à l'ADN sont constitués par des acides nucléiques double brin reproduisant le site de liaison de p53 à l'ADN [El-Deiry et al., Nature 1 (1992) 45 ; Kern et al., Science 252, 1708 ; Friedman et al., PNAS 90 (1993) 3319]. La demanderesse a en effet montré que de tels acides nucléiques étaient capables de complexer les facteurs de transcription présents dans les cellules, de les empêcher de se fixer sur leurs sites endogènes, et ainsi, de bloquer leur activité transcriptionnelle.

Dans un mode préféré, le composé utilisé dans le cadre de la présente invention est un acide nucléique double brin comprenant tout ou partie du site de laison de p53 à l'ADN. Plus préférentiellement, l'acide nucléique comprend tout ou partie de la séquence SEQ ID n° 2 ou un variant actif de celle-ci. Le terme variant actif désigne au sens de l'invention tout variant de la séquence SEQ ID n° 2 ayant conservé les propriétés de fixation à la protéine p53. De tels variants peuvent être obtenus par mutation, délétion, substitution et/ou addition de bases sur la séquence SEQ ID n° 2, puis vérification in vitro de l'activité de liaison.

Dans un autre mode préféré, le composé utilisé dans le cadre de la présente invention est un acide nucléique codant pour une forme mutée de la protéine. p53 capable d'antagoniser l'activité de celle-ci.

Toujours dans un mode préféré, le composé utilisé dans le cadre de la présente invention est un acide nucléique antisens capable de réduire les niveaux d'expression de la protéine p53, au niveau transcriptionnel ou traductionnel. Plus préférentiellement, il s'agit d'un ADN codant pour un acide ribonucléique antisens capable d'inhiber la traduction de l'ARNm cellulaire de p53. Un tel antisens est représenté sur la séquence SEQ ID n° 1.

L'acide nucléique peut être utilisé tel quel, par exemple après injection à l'homme ou l'animal, pour induire une protection ou traiter la dégénérescence neuronale. En particulier, il peut être injecté sous forme d'ADN nu selon la technique décrite dans la demande WO 90/11092. Il peut également être administré sous forme complexée, par exemple avec du DEAE-dextran [Pagano et al., J.Virol. 1 (1967) 891], avec des protéines nucléaires [Kaneda et al., Science 243 (1989) 375], avec des lipides [Felgner et al., PNAS 84 (1987) 7413], sous forme de liposomes [Fraley et al., J.Biol.Chem. 255 (1980) 10431], etc.

Préférentiellement, l'acide nucléique utilisé dans le cadre de l'invention fait partie d'un vecteur. L'emploi d'un tel vecteur permet en effet d'améliorer l'administration de l'acide nucléique dans les cellules à traiter, et également d'augmenter sa stabilité dans lesdites cellules, ce qui permet d'obtenir un effet inhibiteur durable. De plus, il est possible d'introduire plusieurs séquences d'acide nucléique dans un même vecteur, ce qui augmente également l'efficacité du traitement.

Le vecteur utilisé peut être d'origines diverses, dès lors qu'il est capable de transformer les cellules animales, de préférence les cellules nerveuses humaines. Dans un mode préféré de mise en oeuvre de l'invention, on utilise un vecteur viral, qui peut être choisi parmi les adénovirus, les rétrovirus, les virus adéno-associés (AAV), le virus de l'herpès, etc.

A cet égard, la présente invention a également pour objet tout virus recombinant comprenant, inséré dans son génome, un acide nucléique codant pour une forme mutée de la protéine p53 capable d'antagoniser l'activité de celle-ci, et/ou un acide nucléique comprenant tout ou partie du site de laison de p53 à l'ADN et/ou un acide nucléique antisens capable de réduire les niveaux d'expression de la protéine p53, au niveau transcriptionnel ou traductionnel.

Le virus recombinant selon l'invention peut être choisi parmi les adénovirus, les rétrovirus, les virus adéno-associé, etc. De préférence, il s'agit d'un virus capable d'infecter les cellules nerveuses, tel que notamment un adénovirus. Des vecteurs dérivés des adénovirus, des rétrovirus, ou des AAV incorporant des séquences d'acides nucléiques hétérologues ont été décrits dans la littérature [Akli et al., Nature Genetics 3 (1993) 224 ; Stratford-Perricaudet et al., Human Gene Therapy 1 (1990) 241 ; EP 185 573, Levrero et al., Gene 101 (1991) 195 ; Le Gal la Salle et al., Science 259 (1993) 988 ; Roemer et Friedmann, Eur. J. Biochem. 208 (1992) 211 ; Dobson et al., Neuron 5 (1990) 353 ; Chiocca et al., New Biol. 2 (1990) 739 ; Miyanohara et al., New Biol. 4 (1992) 238; WO91/18088].

Avantageusement, le virus recombinant selon l'invention est un virus défectif. Le terme "virus défectif" désigne un virus incapable de se répliquer dans la cellule cible. Généralement, le génome des virus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par l'acide nucléique. Préférentiellement, le virus défectif conserve néanmoins les séquences de son génome qui sont nécessaires à l'encapsidation des particules virales.
Il est particulièrement avantageux d'utiliser les séquences nucléiques de l'invention sous forme incorporée à un adénovirus recombinant défectif.
Il existe en effet différents sérotypes d'adénovirus, dont la structure et les propriétés varient quelque peu, mais qui ne sont pas pathogènes pour l'homme, et notamment les sujets non immuno-déprimés. Par ailleurs, ces virus ne s'intègrent pas dans le génome des cellules qu'ils infectent, et peuvent incorporer des fragments importants d'ADN exogène. Parmi les différents sérotypes, on préfère utiliser dans le cadré de la présente invention les adénovirus de type 2 ou 5 (Ad 2 ou Ad 5). Dans le cas des adénovirus Ad 5, les séquences nécessaires à la réplication sont les régions E1A et E1B.
Un mode de réalisation particulier de l'invention consiste dans un vecteur, notamment viral, comprenant au moins deux acides nucléiques tels que définis ci-dessus.

Les virus recombinants défectifs de l'invention peuvent être préparés par recombinaison homologue entre un virus défectif et un plasmide portant entre autre la séquence d'acides nucléiques telle que définie ci-avant [Levrero et al., Gene 101 (1991) 195 ; Graham, EMBO J. 3(12) (1984) 2917]. La recombinaison homologue se produit après co-transfection desdits virus et plasmide dans une lignée cellulaire appropriée. La lignée cellulaire utilisée doit de préférence (i) être transformable par lesdits éléments, et (ii), comporter les séquences capables de complémenter la partie du génome du virus défectif, de préférence sous forme intégrée pour éviter les risques de recombinaison. A titre d'exemple de lignée utilisable pour la préparation d'adénovirus recombinants défectifs, on peut mentionner la lignée de rein embryonnaire humain 293 [Graham et al., J. Gen. Virol. 36 (1977) 59] qui contient notamment, intégrée dans son génome, la partie gauche du génome d'un adénovirus Ad5 (12 %). A titre d'exemple de lignée utilisable pour la préparation de rétrovirus recombinants défectifs, on peut mentionner la lignée CRIP [Danos et Mulligan, PNAS 85 (1988) 6460].
Ensuite, les virus qui se sont multipliés sont récupérés et purifiés selon les techniques classiques de biologie moléculaire.

La présente invention a également pour objet une composition pharmaceutique comprenant au moins un virus recombinant tel que défini ci-dessus.
Les compositions pharmaceutiques de l'invention peuvent être formulées en vue d'une administration par voie topique, orale, parentérale, intranasale, intraveineuse, intramusculaire, sous-cutanée, intraoculaire, etc.
Préférentiellement, les compositions pharmaceutiques contiennent des véhicules pharmaceutiquement acceptables pour une formulation injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.
Les doses d'acides nucléiques (séquence ou vecteur) utilisées pour l'administration peuvent être adaptées en fonction de différents paramètres, et notamment en fonction du mode d'administration utilisé, de la pathologie concernée, de l'acide nucléique à exprimer, ou encore de la durée du traitement recherchée. D'une manière générale, concernant les virus recombinants selon l'invention, ceux-ci sont formulés et administrés sous forme de doses comprises entre 10⁴ et 10¹⁴ pfu/ml, et de préférence 10⁶ à 10¹⁰ pfu/mL Le terme pfu ("plaque forming unit") correspond au pouvoir infectieux d'une solution de virus, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature.

De telles compositions pharmaceutiques peuvent être utilisées chez l'homme, pour le traitement et/ou la prévention des maladies neurodégénératives, et en particulier, pour le traitement et/ou la prévention de la dégénérescence neuronale associée à l'ischémie, l'hypoxie, l anoxie, l'hypoglycémie, les attaques épileptiques ou encore les traumas cérébraux ou spinaux, ou pour le traitement et/ou la prévention de la chorée de Huntington, de la maladie d'Alzheimer, de la maladie de Parkinson ou de la sclérose latérale amyotrophique.

La présente invention sera plus complètement décrite à l'aide des exemples qui suivent, qui doivent être considérés comme illustratifs et non limitatifs.

### Légende des figures

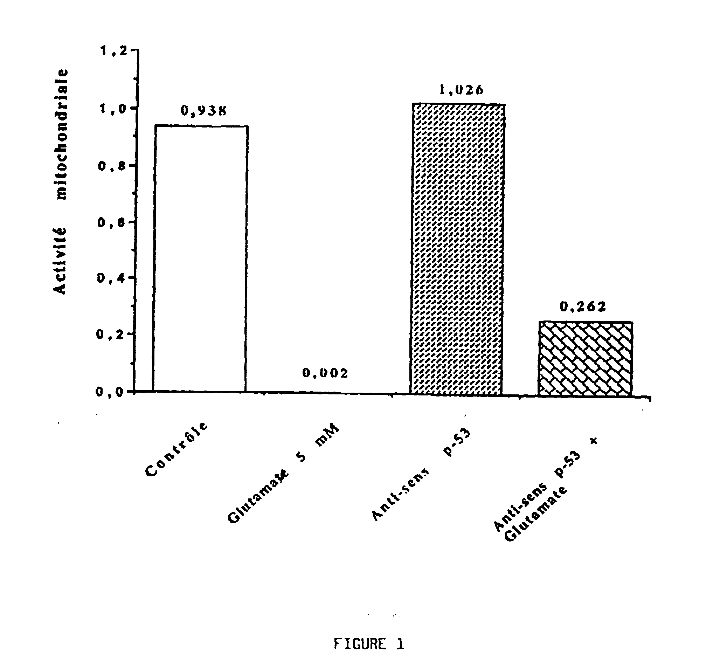

Figure 1 : Inhibition de la mort cellulaire induite par le glutamate sur des cultures primaires de neurones corticaux par un acide nucléique antisens anti-p53.

### Techniques générales de clonage

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982 ; Ausubel F.M. et al. (eds), "Current Protocols in Molecular Biology", John Wiley & Sons, New York, 1987].
Les plasmides de type pBR322, pUC et les phages de la série M13 sont d'origine commerciale (Bethesda Research Laboratories).
Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.
Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.
La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.
L'amplification enzymatique de fragments d'ADN par la technique dite de PCR [Polymérase-catalyzed Chain Reaction, Saiki R.K. et al., Science 230 (1985) 1350-1354 ; Mullis K.B. et Faloona F.A., Meth. Enzym. 155 (1987) 335-350] peut être effectuée en utilisant un "DNA thermal cycler" (Perkin Elmer Cetus) selon les spécifications du fabricant
La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### EXEMPLES

### Exemple 1 : Diminution du volume d'infarct chez les souris ischémiées par suppression du gène p53.

Cet exemple décrit l'effet de la suppression du gène p53 sur le volume d'infarct chez des souris ischémiées. Pour cela, des ischémies ont été induites chez des souris par occlusion de l'artère cérébrale moyenne, et les volumes d'infarct ont été déterminés, puis comparés.
Protocole : Les animaux (souris mâle C57/Blc âgées de 9 à 12 semaines, Genpharm, Danemark ; homozygotes type sauvage ou Δp53) ont été anesthésiés dans un mélange d'oxygène, de protoxyde d'azote et de 1,8 % d'halothane, et maintenus dans ces conditions pendant toute la durée de l'intervention chirurgicale. La température rectale est maintenue à 37°C +/- 0,5 par une couverture chauffante. L'artère cérébrale moyenne gauche a ensuite été cautérisée par électrocoagulation au moyen d'une pince bipolaire. La plaie a ensuite été recousue et les animaux placés dans une pièce à 30°C pendant 24 heures, avec nourriture et boisson ad libitum. Au bout de 24 heures, les animaux ont été sacrifiés par décapitation. Les cerveaux ont été prélevés, immergés dans un bain d'isopentane à -30°C, puis conservés à -80°C. Des coupes histologiques de 40 *µ*m ont ensuite été effectuées dans un cryostat à -20°C, à raison d'une coupe tous les 500 *µ*m, de l'apparition de l'infarctus jusqu'à sa disparition. Ces coupes ont ensuite été colorées au violet de Crésyl. Le volume de l'infarctus est déterminé par analyse d'image. L'analyse statistique est faite à l'aide du test t de Student pour groupes indépendants, après vérification de l'homogénéité des variances. Dans le cas où les variances n'étaient pas homogènes, le test non paramétriques de Wilcoxon a été utilisé.

### Résultats : Les résultats obtenus sont présentés sur le tableau ci après.

| | Souris P53 | Souris Témoin |
|---|---|---|
| Nombre total d'animaux testés | 45 | 46 |
| Poids moyen (g) | 25,73 | 25,44 |
| Température moyenne (°C) | 36,80 | 37,01 |
| Volume moyen d'infarct (mm³) | 24,95 +/-1,81 | 31,54 +/- 1,86 |

Ces résultats font apparaître une diminution de l'ordre de 20 % des volumes d'infarct après ischémie des souris n'exprimant pas le gène p53. Ces résultats démontrent donc qu'une suppression de l'activité p53 permet de ralentir la dégénérescence neuronale.

### Exemple 2 : Inhibition de la mort cellulaire induite par le glutamate sur des cultures primaires de neurones corticaux par un acide nucléique antisens anti-p53

Cet exemple décrit l'effet d'un acide nucléique antisens anti p53 sur la mort induite par le glutamate sur des neurones corticaux de rat embryonnaire, en culture primaire.
Le glutamate est le principal neurotransmetteur excitateur du système nerveux central. Cependant, une exposition au glutamate pendant des périodes anormalement longues, ou à des concentrations plus élevées que les concentrations physiologiques peut provoquer une toxicité neuronale désignée sous le terme d'excitotoxicité [Olney Adv. Exp. Med. Biol. 203 (1986) 631]. De nombreux arguments expérimentaux suggèrent que ce type de toxicité contribue à la dégénérescence neuronale associée à l'ischémie; l'hypoxie, l'hypoglycémie, les attaques épileptiques ou encore aux trauma cérébraux [Choi, J. Neurobiol. 23 (1992) 1261]. L'excitotoxicité serait également impliquée dans la pathogénèse de maladies telles que la chorée de Huntington [Young et al., Science 241 (1988) 981] et la maladie d'Alzheimer [Koh et al., Brain Res. 533 (1990) 315 ; Mattson et al. ; J. Neurosci. 12 (1992) 376]. Cet exemple montre que l'effet toxique du glutamate est inhibé en partie en présence d'un acide nucléique antisens capable de réduire les niveaux d'expression de la protéine p53.
Préparation et séquence de l'acide nucléique antisens : L'oligonucléotide antisens a été synthétisé au moyen d'un synthétiseur automatique de nucléotides (Maniatis). La séquence de l'oligonucléotide est la suivante : 5'-CGACTGTGAATCCTCCAT-3' (SEQ ID n° 1).
Etude de l'inhibition : Les cellules de cortex de rat Wistar embryonnaire (E17) ont été isolées selon la méthode de Dichter [Brain Res. 149 (1978) 279], mises en culture sur boites 6 puits (35 mn ; densité 6.10⁵ cellules/boîte) Costar, dans du milieu DMEM (Dulbecco's Modified Eagle Medium) contenant 10 µg/ml insuline, 10 µg/ml transferrine, 10 ng/ml selenite de sodium, 10 nM progestérone, 1 nM triiodothyronine,
et conservées dans une étuve (37°C, 5 % CO2). 2 µM d'acide nucléique antisens anti-p53 décrit ci-dessus ont ensuite été ajoutés aux cultures, lors de l'ensemencement, puis aux jours 1 et 2. Le glutamate (5 mM) a été administré au jour 2, en même temps que l'acide nucléique antisens anti-p53. La toxicité induite par le glutamate a été déterminée après 24 heures de culture, par mesure de l'activité mitochondriale selon la technique décrite par Manthorpe et al. [Dev. Brain. Res. 25 (1986) 191].

Les résultats obtenus sont présentés sur la figure 1. Ils montrent clairement que l'acide nucléique antisens anti-p53 est capable de réduire d'environ 25 % la toxicité induite par le glutamate.

## Revendications

1. Utilisation d'un composé inhibant au moins partiellement l'activité de la protéine p53 pour la préparation d'une composition pharmaceutique destinée au traitement et/ou à la prévention des maladies neurodégénératives.

2. Utilisation selon la revendication 1 **caractérisée en ce qu'**il s'agit d'un composé agissant sur la synthèse de la protéine p53, aux niveaux transcriptionnel, traductionnel ou post-traductionnel, et/ou sur la liaison de p53 à l'ADN.

3. Utilisation selon la revendication 2 **caractérisée en ce que** le composé est un acide nucléique double brin comprenant tout ou partie du site de laison de p53 à l'ADN.

4. Utilisation selon la revendication 2 **caractérisée en ce que** le composé est un acide nucléique codant pour une forme mutée de la protéine p53 capable d'antagoniser l'activité de celle-ci.

5. Utilisation selon la revendication 2 **caractérisée en ce que** le composé est un acide nucléique antisens capable de réduire les niveaux d'expression de la protéine p53, au niveau transcriptionnel ou traductionnel.

6. Utilisation selon la revendication 5 **caractérisée en ce que** l'acide nucléique antisens est un ADN codant pour un acide ribonucléique antisens capable d'inhiber la traduction de l'ARNm cellulaire de p53.

7. Utilisation selon les revendications 3 à 5 **caractérisée en ce que** l'acide nucléique fait partie d'un vecteur.

8. Utilisation selon la revendication 7 **caractérisée en ce que** l'acide nucléique fait partie d'un vecteur viral.

9. Utilisation selon la revendication 8 **caractérisée en ce que** le vecteur viral comprend, inséré dans son génome, au moins un acide nucléique codant pour une forme mutée de la protéine p53 capable d'antagoniser l'activité de celle-ci, et/ou un acide nucléique comprenant tout ou partie du site de laison de p53 à l'ADN et/ou un acide nucléique antisens capable de réduire les niveaux d'expression de la protéine p53, au niveau transcriptionnel ou traductionnel.

10. Utilisation selon la revendication 9 **caractérisé en ce que** le vecteur est un adénovirus, un rétrovirus, un virus adéno-associé, ou un virus de l'herpès.

11. Utilisation selon la revendication 10 **caractérisé en ce qu'**il s'agit d'un adénovirus.

12. Utilisation selon l'une des revendications 7 à 10 **caractérisé en ce que** l'acide nucléique comprend tout ou partie de la séquence SEQ ID n° 2 ou de variants actifs de celle-ci.

13. Utilisation selon l'une des revendications 9 à 12 **caractérisé en ce** le virus comprend plusieurs acides nucléiques identiques ou différents, tels que définis dans les revendications 3 à 5.

14. Utilisation selon l'une des revendications 9 à 13 **caractérisé en ce** le virus est d'un virus défectif.

## Patentansprüche

1. Verwendung einer Verbindung, die mindestens teilweise die Aktivität von Protein p53 inhibiert, zur Herstellung einer pharmazeutischen Zusammensetzung, die für die Behandlung und/oder Vorbeugung von neurodegenerativen Erkrankungen vorgesehen ist.

2. Verwendung nach Anspruch 1, **dadurch gekennzeichnet, daß** es sich um eine Verbindung handelt, die auf die Synthese von Protein p53 im transkriptionellen, translationellen oder post-translationellen Bereich und/oder auf die Bindung von p53 an die DNA einwirkt.

3. Verwendung nach Anspruch 2, **dadurch gekennzeichnet, daß** die Verbindung eine doppelsträngige Nucleinsäure ist, umfassend ganz oder teilweise Bereiche der Bindung von p53 an die DNA.

4. Verwendung nach Anspruch 2, **dadurch gekennzeichnet, daß** die Verbindung eine Nucleinsäure ist, die für eine mutierte Form von Protein p53 kodiert, die fähig ist, dessen Aktivität zu antagonisieren.

5. Verwendung nach Anspruch 2, **dadurch gekennzeichnet, daß** die Verbindung eine Antisens-Nucleinsäure ist, die fähig ist, die Bereiche der Expression von Protein p53 im transkriptionellen oder translationellen Bereich zu reduzieren.

6. Verwendung nach Anspruch 5, **dadurch gekennzeichnet, daß** die Antisens-Nucleinsäure eine DNA ist, die für eine Antisens-Ribonucleinsäure kodiert, die fähig ist, die Translation von zellulärer RNAm von p53 zu inhibieren.

7. Verwendung nach Anspruch 3 bis 5, **dadurch gekennzeichnet, daß** die Nucleinsäure den Teil eines Vektors bildet.

8. Verwendung nach Anspruch 7, **dadurch gekennzeichnet, daß** die Nucleinsäure den Teil eines viralen Vektors bildet.

9. Verwendung nach Anspruch 8, **dadurch gekennzeichnet, daß** der virale Vektor, eingeordnet in seinem Genom, mindestens eine Nucleinsäure umfaßt, die für eine mutierte Form von Protein p53 kodiert, die fähig ist, dessen Aktivität zu antagonisieren, und/oder eine Nucleinsäure, die ganz oder teilweise Bereiche der Bindung von p53 an die DNA umfaßt, und/oder eine Antisens-Nucleinsäure, die fähig ist, die Bereiche der Expression von Protein p53 im transkriptionellen oder translationellen Bereich zu reduzieren.

10. Verwendung nach Anspruch 9, **dadurch gekennzeichnet, daß** der Vektor ein Adenovirus, ein Retrovirus, ein adeno-assoziierter Virus oder ein Herpesvirus ist.

11. Verwendung nach Anspruch 10, **dadurch gekennzeichnet, daß** es sich um einen Adenovirus handelt.

12. Verwendung nach einem der Ansprüche 7 bis 10, **dadurch gekennzeichnet, daß** die Nucleinsäure ganz oder teilweise die Sequenz SEQ ID No. 2 oder aktive Varianten davon umfaßt.

13. Verwendung nach einem der Ansprüche 9 bis 12, **dadurch gekennzeichnet, daß** der Virus mehrere identische oder unterschiedliche Nucleinsäuren umfaßt, wie in Anspruch 3 bis 5 definiert.

14. Verwendung nach einem der Ansprüche 9 bis 13, **dadurch gekennzeichnet, daß** der Virus ein defektiver Virus ist.

## Claims

1. Use of a compound that at least partially inhibits the activity of the p53 protein, for preparing a pharmaceutical composition intended for the treatment and/or for the prevention of neurodegenerative diseases.

2. Use according to Claim 1, **characterized in that** the compound is a compound that acts on the synthesis of the p53 protein, at the transcriptional, translational or posttranslational level, and/or on the binding of p53 to DNA.

3. Use according to Claim 2, **characterized in that** the compound is a double-stranded nucleic acid comprising all or part of the DNA-binding site of p53.

4. Use according to Claim 2, **characterized in that** the compound is a nucleic acid encoding a mutated form of the p53 protein capable of antagonizing the activity thereof.

5. Use according to Claim 2, **characterized in that** the compound is an antisense nucleic acid capable of reducing the expression levels of the p53 protein, at the transcriptional or translational level.

6. Use according to Claim 5, **characterized in that** the antisense nucleic acid is a DNA encoding an antisense ribonucleic acid capable of inhibiting the translation of the p53 cellular mRNA.

7. Use according to Claims 3 to 5, **characterized in that** the nucleic acid is part of a vector.

8. Use according to Claim 7, **characterized in that** the nucleic acid is part of a viral vector.

9. Use according to Claim 8, **characterized in that** the viral vector comprises, inserted into its genome, at least one nucleic acid encoding a mutated form of the p53 protein capable of antagonizing the activity thereof, and/or a nucleic acid comprising all or part of the DNA-binding site of p53, and/or an antisense nucleic acid capable of reducing the expression levels of the p53 protein, at the transcriptional or translational level.

10. Use according to Claim 9, **characterized in that** the vector is an adenovirus, a retrovirus, an adeno-associated virus or a herpesvirus.

11. Use according to Claim 10, **characterized in that** it involves an adenovirus.

12. Use according to one of Claims 7 to 10, **characterized in that** the nucleic acid comprises all or part of the sequence SEQ ID No. 2 or of active variants thereof.

13. Use according to one of Claims 9 to 12, **characterized in that** the virus comprises several identical or different nucleic acids, as defined in Claims 3 to 5.

14. Use according to one of Claims 9 to 13, **characterized in that** the virus is a defective virus.


### LISTE DE SEQUENCES

(1) INFORMATION GENERALE:
   (i) DEPOSANT:
      (A) NOM: RHONE-POULENC RORER S.A.
      (B) RUE: 20, avenue Raymond ARON
      (C) VILLE: ANTONY
      (E) PAYS: FRANCE
      (F) CODE POSTAL: 92165
   (ii) TITRE DE L' INVENTION: Compositions pharmaceutiques et leur utilisation, notamment pour le traitement des maladies neurodegeneratives.
   (iii) NOMBRE DE SEQUENCES: 2
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADNc
   (iii) HYPOTHETIQUE: NON
   (iii) ANTI-SENS: OUI
   (xi) DESCRIPTION DE LA SEQUENCE: SEQ ID NO: 1:
   CGACTGTGAA TCCTCCAT 18
(2) INFORMATION POUR LA SEQ ID NO: 2:
   (i) CARACTERISTIQUES DE LA SEQUENCE:
      (A) LONGUEUR: 20 paires de bases
      (B) TYPE: acide nucléique
      (C) NOMBRE DE BRINS: double
      (D) CONFIGURATION: linéaire
   (ii) TYPE DE MOLECULE: ADNc
   (iii) HYPOTHETIQUE: NON
   (iii) ANTI-SENS: NON
   (xi) DESCRIPTION DE LA SEQUENCE: SEQ ID NO: 2:
   GGACATGCCC GGGCATGTCC 20